# EUROPÄISCHE PATENTSCHRIFT

(11) **EP 2 032 179 B1**
(45) Veröffentlichungstag und Bekanntmachung des Hinweises auf die Patenterteilung: **05.10.2011**
(21) Anmeldenummer: 07765600.7
(22) Anmeldetag: 25.06.2007
(51) Int. Cl.: A61L 15/22, A61L 15/60, A61L 15/44

(54) **WUNDAUFLAGE**
WOUND DRESSING
PANSEMENT

(30) Priorität: 29.06.2006 EP 06116313; 29.06.2006 EP 06116317
(43) Veröffentlichungstag der Anmeldung: 11.03.2009
(73) Patentinhaber: BioCell Gesellschaft für Biotechnologie mbH, 51674 Wiehl (DE)
(72) Erfinder: HORCHLER, Daniela, 51674 Wiehl (DE); HORCHLER, Harald K., 51674 Wiehl (DE)
(74) Vertreter: von Kreisler Selting Werner
(86) Internationale Anmeldenummer: PCT/EP2007/056321
(87) Internationale Veröffentlichungsnummer: WO 2008/000720

(56) Entgegenhaltungen:
- WO-A-02/066085
- WO-A-03/053484

## Beschreibung

Die vorliegende Erfindung betrifft eine Wundauflage und ihre Verwendung.

Im Stand der Technik sind eine Vielzahl von Wundauflagen bekannt, die primär dazu dienen, das Eindringen von Fremdkörpern in eine Wunde zu verhindern und Blut- oder Wundsekret aufzusaugen.

Unterschieden wird zwischen der trockenen Wundbehandlung und der feuchten Wundbehandlung.

Die trockene Wundbehandlung dient im wesentlichen dazu, kleinere Verletzungen zu schützen und Wundsekret aufzusaugen.

In der feuchten Wundbehandlung wird versucht, ein verbessertes Wundheilungsmilieu durch den Schutz der Wunde vor Austrocknung und Eindringen von Keimen zu schaffen, wobei möglichst ein Austausch von Gasen und Wasserdampf gewährleistet sein soll. Solche feuchte Wundbehandlung wird für sekundär heilende Wunden, chronische Wunden und gelegentlich auch bei primär heilenden Wunden eingesetzt.

Bei der sekundären Wundheilung besteht ein Gewebedefizit, das zu klaffenden Wundrändern führt, die nicht durch Nähen, Klammern oder ähnliches zu verschließen sind. Erforderlich ist, die Proliferation des körpereigenen Gewebes zu fördern, um die Wunde abzudecken.

Es sind verschiedene Materialien im Einsatz, mit denen versucht wird, verbesserte Wundverbände herzustellen. Solche auch hydroaktiven Verbände genannten Materialien bestehen beispielsweise aus Alginaten, aus Polymerschäume auf Polyurethanbasis oder Faserfilamenten, wie beispielsweise Carboxymethylcellulose. Teilweise sind diese mit Silberionen dotiert, um eine bakterizide Wirkung zu erzielen. Grad und Menge der aufgenommenen Feuchtigkeit, die definierte Silberionenfreisetzung und ähnliche Parameter definieren die Leistungsfähigkeit der Produkte.

Beispielsweise offenbart die WO 03/053484 A1 eine hydrophile Textilmatrix, die eine antibiotische Aktivität aufweist. In der WO 02/066085 A1 wird eine Wundauflage mit einer aktivkohlehaltigen Schicht offenbart, wobei die Wundauflage eine weitere Schicht mit einem antimikrobiell wirksamen Mittel enthält.

Die bisher im Markt erhältlichen Produkte sind alle nicht zufriedenstellend, teilweise sind die Materialien nicht stabil genug, dass heißt nach entsprechender Feuchtigkeitsaufnahme können sie nicht ihre Konsistenz bewahren. Teilweise sind die Silberionenfreisetzungen zu groß, so dass es im Gewebe zu Silberchloridausfällungen kommt.

Gegenstand der Erfindung ist daher eine Wundauflage, die zumindest einige der Nachteile des Stand der Technik überwindet.

Die erfindungsgemäße Wundauflage weist eine Absorberschicht aus einem Funktionsvlies umfassend mindestens erste und zweite Filamente,
wobei die ersten Filamente einen Kern aus einem Polyethylenterephtalat und einen Mantel aus Polyolefin umfassen und im Mantel eine Silberquelle eingebunden ist und
wobei die zweiten Filamente einen Kern aus Polyacrylnitril und einen Mantel aus Polyacryl umfassen,
wobei die Absorberschicht in der Lage ist, 0,15 bis 1,20 ml/cm² Wasser zu absorbieren, auf.

In einer bevorzugten Form weist der Wundverband mindestens zwei Schichten auf, wobei die eine Schicht eine wundnahe Schicht aus einem hydrophoben Material mit einer lichten Öffnungsweite von 10 bis 25 µm ist.
Hydrophob im Sinne dieser Anmeldung sind solche Materialien, die einen Kontaktwinkel von mehr als 90° mit Wasser bilden. Geeignete Materialien sind insbesondere Polyolefine, beispielsweise Polyethylene.

Die wundnahe Schicht weist eine lichte Öffnungsweite von 10 bis 25 µm auf, dass heißt die Schicht ist aufgrund der Poren durchlässig. Die lichte Öffnungsweite ist - unabhängig von der exakten geometrischen Form der Öffnung - die größte Entfernung zwischen zwei Punkten. Soweit das Material unterschiedliche große Poren aufweist, ist die definierte lichte Öffnungsweite die mittlere lichte Öffnungsweite der Poren. Solche lichten Öffnungsweiten in dieser Größenordnung lassen sich unproblematisch mittels eines Mikroskops bestimmen und auswerten.

Bestandteil der Wundauflage ist eine Absorberschicht aus einem Funktionsvlies. Diese Funktionsvlies umfasst mindestens zwei Filamente. Das erste Filament weist einen Kern aus einem Polyethylenterephthalat auf und einen Mantel aus einem Polyolefin auf. In diesem Mantel ist eine Silberquelle eingebunden, die in der Lage ist, Silberionen freizusetzen.

Geeignete Polyolefine für den Mantel sind beispielsweise Polyethylen, Polypropylen. Die Begriffe Polyethylen und Polypropylen umfassen Varianten wie HDPE, MDPE, LDPE, LLPE, orientierte und biaxial orientierte Polypropylene, (OPP und BOPP).

Zur Silberionenfreisetzung enthält das erste Filament bevorzugt Silberzeolithe, der in dem äußeren Mantel eingeschmolzen und dadurch fest eingebunden ist. Geeignete Konzentrationen an Silberzeolith liegen in der Größenordnung von 0,8 bis 5 Gew.-%, bevorzugt 1,5 bis 5 Gew.-% oder 0,8 bis 1,5 Gew.-%

Als zweites Filament werden Filamente eingesetzt, die einen Kern aus Polyacrylnitril haben und einen Mantel aus superabsorbierendem Polyacryl. Die Absorberschicht weist außen ein superabsorbierendes Polyacrylmaterial auf, das bevorzugt aus einem Copolymer aus Polyacrylsäure und Polyammoniumacrylat besteht.

Die Absorberschicht ist in der Lage zwischen 0,15 und 1,2 ml Wasser pro cm² in der Absorberschicht zu absorbieren. Die Messung der Absorption von Verbandmitteln ist in der DIN EN ISO 13726-1 beschrieben.

In einer Ausführungsform ist die Absorberschicht wiederum aus mehreren Einzelschichten aufgebaut. Die einzelnen Schichten sind als Stapelfasern hochdichtvernadelt und können verschiedene Flächengewichte aufweisen. Das Gesamtflächengewicht der Absorberschicht liegt bevorzugt zwischen etwa 150 und 350 g/m², die sich auf die mehreren Schichten verteilen können.

In einer Ausführungsform der Erfindung bilden die mindestens zwei Filamente der Absorberschicht zusammen eine Helix; diese wirkt sich positiv auf die Feuchtigkeitsführung aus.

In einer weiteren bevorzugten Ausführungsform ist die Absorberschicht in der Lage, im Kontakt mit Wasser über einen Zeitraum von 72 Stunden zwischen 10 und 100 µg Silberionen pro 100 cm² der Wundauflage abzugeben. Die Testbedingungen sind im Beispiel beschrieben. Hierdurch wird zum einen erreicht, dass diese Konzentration über diesen Zeitraum so hoch ist, dass sie wirksam ist. Auf der anderen Seite wird verhindert, dass sie zu hoch ist, wobei beispielsweise Silberchlorid in der Wunde ausfällt.

Der geeignete Durchmesser für die Filamente liegen zwischen 5 und 10 µm, wobei in einer Ausführungsform der Kern bevorzugt einen Durchmesser in der Größenordnung von 0,5 bis 2 µm aufweist. Durch den Einsatz von Filamenten mit einem Kern und einem Mantel werden vorteilhafte Eigenschaften der jeweiligen Materialien kombiniert.

In einer Ausführungsform sind die wundnahe Schicht und die Absorberschicht mit einem Klebstoff verbunden.

In einer bevorzugten Ausführungsform ist die Wundauflage besonders schnell aufsaugend. Die Absorberschicht kann dann innerhalb von 10 Sekunden 50-70% ihrer Absorbtionskapazität aufnehmen. Vorteilhaft ist weiterhin, wenn die Wundauflage unter Druck nur einen geringen Anteil der Aufnahme der Flüssigkeit abgibt. Bevorzugt behält die Wundauflage bei einem Druck von 3.500 Pa (20 mm Hg) mindestens 50% des aufgenommenen Wassers. Der Test ist im Beispiel beschrieben.

Weiterhin ist es vorteilhaft, dass die Wundauflage eine geringe Querfeuchtigkeit aufweist. Querfeuchtigkeit bedeutet, dass eine Befeuchtung einer Stelle nicht dazu führt, dass sich die Flüssigkeit im gesamten Material verteilt. Das hat den Vorteil, dass hierdurch Kontamination zwischen den verschiedenen Bereichen der Wunden verhindert werden. Bevorzugt liegt die Querfeuchtigkeit unter 15 mm pro 24 Stunden. Der Test ist in der DIN EN ISO 13726-2 beschrieben.

Bevorzugt ist es weiterhin so, dass im zweiten Filament bei Kontakt mit Wasser die Faser nicht ihre Konsistenz verliert, dass heißt, dass sich der äußere Mantel nicht vom Kern ablöst. Dies wird dadurch definiert, dass der Durchmesser der Faser im Wasser maximal auf das Doppelte ansteigt und makroskopisch keine Ablösung der Schicht zu erkennen ist.

Die vorhandenen Filamente müssen nicht im gleichen Verhältnis in der Absorberschicht enthalten sein. Typischerweise liegt das Gewichtsverhältnis des ersten und zweiten Filaments der Absorberschicht zwischen 90: 10 und 50:50, bevorzugt zwischen 90:10 und 70:30.

Die Erfindung erlaubt weiterhin die Verwendung der Wundauflage zur Behandlung von Wunden, insbesondere von chronischen Wunden, wie sie durch verminderte Mikrozirkulation und verminderten Sauerstoffsättigungskoeffizienten, freie Radikale oder Druckischämie hervorgerufen werden.

Besonders bevorzugte Einsatzgebiete für die erfindungsgemäße Wundauflage sind Druckgeschwüre (Dekubitus), arterielles Ulcus, wie es bei peripherer arterieller Verschlusskrankheit auftreten kann, venöser Ulcus, wie es bei chronisch venöser Insuffizienz auftreten kann, neuropathischer Ulcus, beispielsweise durch diabetisches Fußsyndrom, bei Verbrennungen, insbesondere Grad IIa/IIb, durch Post OP Wunden, radiologische Wunden (Strahlenulcera), wie sie beispielsweise bei Strahlenbehandlungen nach Tumorresektion auftreten. Ein weiteres Einsatzgebiet ist die Behandlung von Bakterien verursachten Wunden und chemisch verursachten Wunden, insbesondere auch durch biologische oder chemische Waffen.

Das erfindungsgemäße Material kann auch als Feuchtelektrode verwendet werden. Beispielsweise kann die Wundauflage mit einer Elektrolytlösung hydriert werden um dann mittels einer Elektrode Gleichstrom auf die Wundfläche zu übertragen, bevorzugt einen monophasischen Doppelamplituden-Gleichstrom. Es ist beschrieben worden, das hierdurch die Bildung von Vascular Epithelial Growth Factor (VEGF) gefördert wird.

Bevorzugt liegt die Behandlungszeit in einem Bereich von 15 min. bis 24 Stunden mit einer Pulsrate von 100 bis 120 Pulsen pro Sekunde. Bevorzugt wird ein niedrig gepulster Gleichstrom eingesetzt mit einer Spannung von bis zu 200 Volt. Die Versorgung einer solchen Einheit kann beispielsweise auch durch Batterien erfolgen. Die typische Kurvenform des Gleichstroms ist ein monophasischer Exponential-Gleichstromimpuls mit einem fixen Pulsinterval von 100 µs.

Die Wundauflage kann auch als Waschtuch eingesetzt werden, um Wunden zu reinigen. Die Freisetzung von Silberionen ermögticht dadurch bakterizide Wirkung auf die Umgebung. Das Materialgewicht liegt bevorzugt im Bereich von 40 bis 100 g/m².

Alternativ kann die Wundauflage auch als Waschhandschuh und/oder als Waschbett eingesetzt werden.

In einer weiteren Ausführungsform kann die Wundauflage als Pad in eine Waschmaschine gegeben werden, beispielsweise indem sie in einen wasserdurchlässigen Beutel eingeschlossen wird. Sie emittiert hierbei Silberionen, um eine Biofilmbildung in der Waschmaschine zu verhindern. Hierfür genügt bereits eine Konzentration im Bereich von 10⁻⁶ bis 10⁻⁹ mol/l pro Liter in der Waschlösung.

Gegenstand der Erfindung ist weiterhin ein Kit, das zusätzlich ein Hyaluronsäurespray enthält. Das erfindungsgemäße Kit weist in Kombination mit Hyaluronsäure besonders gute Wirksamkeit auf.

Das Hyaluronsäurespray hat bevorzugt folgende Eigenschaften:
- 1 bis 5 mg/ml nicht-quervernetzte Hyaluronsäure
- Puffersubstanzen und Salze, um
   - einen pH-Wert von 5,5- 6,0 und
   - eine Osmolalität von 250 bis 350 mOsmol/kg einzustellen;
- Wasser.

Das Spray enthält 1 bis 5 mg/ml nicht quervernetzte Hyaluronsäure. Bevorzugt wird eine Menge zwischen 1 und 3 mg/ml. Zusätzlich enthält das Spray Puffersubstanzen und Salze, um den pH-Wert auf den Bereich von 5,5 bis 6,5 und die Osmolalität auf einen Bereich von 250 bis 350 mOsmol/kg einzustellen.

Die Osmolalität ist die Anzahl der gelösten Teilchen pro Kilogramm Lösung. Beispielsweise werden aus einem Mol Natriumchlorid durch Auflösen im Wasser aufgrund der Dissoziation zwei Osmol Teilchen.

Weiterhin enthält das Wundspray Wasser.

Geeignete, nicht-quervernetzte Hyaluronsäure hat bevorzugt ein mittleres Molekulargewicht im Bereich von 400 bis 600 kDa. In einer bevorzugten Ausführungsform handelt es sich um eine Hyaluronsäure, die durch Biofermentation hergestellt wird und nicht - wie häufig im Stand der Technik - aus Hahnenkämmen isoliert wird. Biofermentierte Hyaluronsäure ist frei von allergenen Verunreinigungen, wie sie häufig in Produkten aus Hahnenkämmen enthalten sind. Ein besonders geeigneter Stamm zur Herstellung der Hyaluronsäure ist *staphylococcus pyogenes* H4489A. Die Hyaluronsäure kann auch als Salz, z.B. Na- oder K-Salz eingesetzt werden.

Als Puffersubstanzen eignen sich biokompatible, nicht-toxische Komponenten, beispielsweise Natriumchlorid, Kaliumchlorid, Calciumchlorid. Die Einstellung des pH-Wertes kann zum Beispiel durch Pufferung mit Natriumhydrogencarbonat erfolgen.

Eine besonders bevorzugte Zusammensetzung entspricht der bekannten Ringerlösung und enthält 9 g/l NaCl, 0,2 g/l KCI, 0,2 g/l CaCl₂ und 0,1 g/l NaHCO₃.

In dem erfindungsgemäßen Wundspray können weitere Substanzen enthalten sein, wie Mittel zur Einstellung der Viskosität.

In einer bevorzugten Ausführungsform enthält das Wundspray keine weiteren Wirkstoffe. Wirkstoffe im Sinne dieser Anmeldung sind Substanzen, die eine Wirkung über Rezeptoren von Patientenzellen hervorrufen, insbesondere Stoffe auf Peptidbasis, wie Wachstumsfaktoren.

In einer weiteren bevorzugten Ausführungsform der Erfindung ist das Spray frei von Konservierungsstoffen. Hier hat sich gezeigt, dass Konservierungsstoffe negative Auswirkungen auf die Wundheilung haben können.

Erfindungsgemäß wird das Spray eingesetzt, um damit die Wunde im gesamten Bereich einzusprühen. Um eine geeignete Dosierung zu erreichen, sollten die Spraymengen nur gering sein. Eine Menge pro Sprüh-Hub in der Größenordnung von 0,1 bis 0,15 ml ist besonders geeignet.

Eine geeig18e Viskosität liegt im Bereich von 1 bis 8,5 m²/sec. Die Viskosität führt dazu, dass das Spray noch so dünnflüssig ist, dass gut gesprüht werden kann und es sich gut verteilt, auf der anderen Seite aber auch nicht zu dünnflüssig ist und daher sofort von der Wunde abfließt.

Die aufgesprühte Menge an Hyaluronsäure trocknet in kurzer Zeit an und kann durch seine weiteren Bestandteile das Gewebe mit wichtigen Nährstoffen, beispielsweise Salzen, versorgen.

Die erfindungsgemäße Verabreichung des Sprays führt zu einem besonders problemlosen, für den Patienten schmerzfreien Auftragen, da im Gegensatz zu üblichen Kompressen etc. kein Druck auf die Wunde ausgeübt wird. Insbesondere ist vorteilhaft, dass die eingesetzte Hyaluronsäure vom Körper absorbiert werden kann, dass heißt, dass es nicht erforderlich ist, den entstandenen Wundverband von der Wunde wieder abzulösen.

Überraschenderweise zeigt sich, dass durch das erfindungsgemäße Spray die Phagozytose, die Fibroblastenprofileration, die Freisetzen von Cytokin, die Zellmigration, die Angiogenese und die Kollagensynthese gesteigert werden konnten.

Die Wundauflage und insbesondere das Kit eignen sich auch zur Behandlung von hypertrophem Gewebe und cortisongeschädigter Haut.

Die Erfindung wird durch die nachfolgenden Beispiele näher erläutert:

### Beispiel 1

Polyethylenterephthalat und Polyethylen werden eingeschmolzen und zu dem Polyethylen wird ein Silberzeolith in einer Menge von 2,5 Gew.-% gegeben. Es erfolgt eine Coextrusion der beiden Polymere, wobei das Polyethylenterephthalat den Kern bildet. Durch einen Temperatursturz und Extension wird ein Stärke von 2,9 dtex eingestellt.

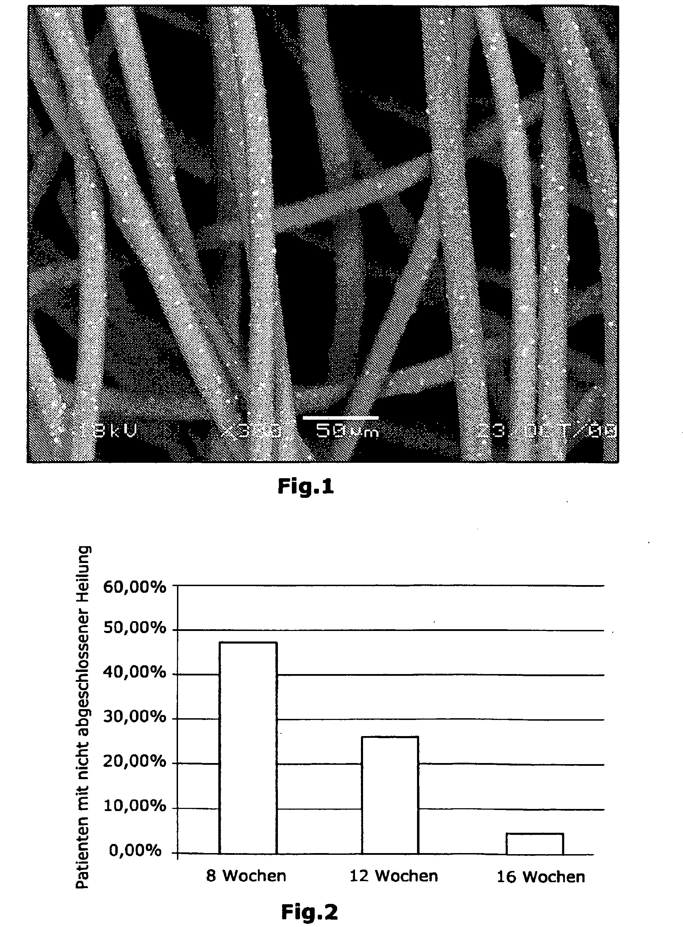

Figur 1 zeigt eine REM-Aufnahme der Faser.

Das zweite Filament wird hergestellt durch Coextrusion von Polyacylnitril und einem Polyacylsäure/Polyammoniumacrylat-Copolymer.

Aus dem ersten und dem zweiten Filament werden Bruchstücke von 51 mm Länge erzeugt, im Gewichtsverhältnis 30:70 vermischt und mit Überschallgeschwindigkeit auf ein laufendes Gummiwalzwerk aufgeschossen. Hierdurch entsteht ein non-woven Stapelvlies.

Drei Lagen (25 g/m², 220 g/m² und 25 g/m²) des oben genannten Materials werden übereinander gelegt und durch "needle punch" verbunden. Die entstandene Textilfläche wird mit einem hypoallergenen Akrylatkleber beschichtet und mit einem Polyolefin thermisch verklebt. Das Produkt wird dann geschnitten und verpackt. Eine abschließende Sterilisation erfolgt mit ionisierender Strahlung von ⁶⁰Co.

### Beispiel 2: Untersuchung der Eigenschaften

### Beispiel 2a: Messung der Silberfreisetzung

Die Silberionenfreisetzung wurde mittels ICP-OES Analyse (Inductively Cuppled Plasma Emission Spectroscopy) gemessen. Die Freisetzungsrate betrugt 60,15 µg/100cm².

### Beispiel 2b: Messung des Adsorptionsverhalten

Die Messung des Absorptionsverhaltens erfolgte nach DIN EN 13726-1 - Absorption; das Prüfverfahren bestimmt die Beurteilung der Saugeigenschaften einer Wundauflage unter Beachtung der Saugleistung plus Transport des Feuchtdampfes bei Kontakt mit der Flüssigkeit. Es ergab sich ein Absorptionsvermögen von 77,55 g/100 cm².

### Beispiel 2c: Bestimmung der Querfeuchtigkeit

Die Messung der Querfeuchtigkeit erfolgte nach DIN EN 13726-2 - Feuchtigkeitsdurchdringungsrate; das Prüfverfahren bestimmt die Beurteilung der Feuchtigkeitstransportrate durchlässiger Folienverbindungen. Es wurden 11 mm/24 h gemessen.

### Beispiel 2d: Messung des Verhaltens unter Kompressionsdruck

Die Messung erfolgte gemäß DIN EN 13726.3 - Wasserdichtigkeit; das Prüfverfahren bestimmt die Beurteilung der Fähigkeit, für die Dauer von 300s einem hydrostatischem Druck von 500 mm Wassersäule Stand zu halten. Hierbei konnten etwa 70% der Kapazitätsmenge gehalten werden. Bei einem Test mit einem Druck von 26,66 mbar (20 mm Hg) konnten 18 g/100 cm² gehalten werden.

### Beispiel 2e: Mechanische Eigenschaft

Das Produkt hatte ein Gesamtgewicht von etwa 290 g/m² und eine Dicke von 2,5mm. Die Höchstzugkraft links (EN 29073-03) betrugt 31 n/50 mm, die Dehnung längs gemäß EN 29073-03 9%.

### Beispiel 3:

Hyaluronsäurenatriumsalz, medical grade, gewonnen durch Biofermentation wurde einer Qualitätsprüfung unterzogen. Das Produkt hatte ein Molekulargewicht zwischen 400 und 600 kDa. Der Gehalt an Protein lag s 0,3 Gew.-%, an Chlorid s 0,5 Gew.-% und an Eisen ≤ 80 ppm.

Es wurden 2 g Hyaluronsäurenatriumsalz in einem Liter Ringerlösung gelöst. Die Ringerlösung enthielt 9 g Natriumchlorid, 0,2 g Kaliumchlorid, 0,2 g Calciumchlorid, 0,1 g Natriumhydrogencarbonat auf 1 I. Der pH-Wert wurde geprüft und mit Natronlauge bzw. Salzsäure auf einen Bereich von 6,0 eingestellt. Es stellte sich eine Osmolalität von etwa 200 mOsmol/kg ein.

Das fertige Produkt wurde in eine Sprühvorrichtung (Comod^{®}-System) eingefüllt.

Das erfindungsgemäße Produkt kann direkt auf die gereinigte Wunde gesprüht werden. Abhängig von der Wundgröße genügen ca. zwei Sprühhübe.

### Beispiel 4:

### Anwendungsbeobachtung

Es wurde eine vierwöchige kontrollierte Anwendungsbeobachtung mit der erfindungsgemäßen Wundauflage an 10 Patienten mit chronisch venöser Insuffizienz (CVI) bei drei bis vier Verbandwechseln je Woche durchgeführt. Die Behandlung erfolgte als Feuchtebehandlung. Bei nicht hinreichender Eigenhydrierung wurde mit Ringerlösung kompensiert. Alle Patienten wurden mit einem Dokumentationssystem erfasst und protokolliert. Die Patienten waren nicht speziell selektiert, sie entsprachen dem normalen Patientenklientel einer Schwerpunktpraxis zur Ulcus-Behandlung. Dabei wurde die Wundbeurteilung, - interpretation analog Falanga T.I.M.E. vorgenommen.

**Tabelle 1 zeigt wesentliche Ergebnisse der Anwendungsbeobachtung bei 10 Patienten.**

| ALLGEMEINE ERGEBNISGRUNDLAGEN | |
|---|---|
| Bei 10 Patienten durchgeführte Verbandwechsel | 130 |
| Kumulierte Behandlungs- /Heilungstage | 327 Tage |
| Kumulierte Wundflächengrößen | 42,2 cm² |
| Kumulierte Restflächengrößen | 17,6 cm² |
| Gesamtaufwand aller Materialkosten (AEP brutto) | 1.020,00 € |

| KLINISCHE DATEN | |
|---|---|
| Wundflächenreduzierung in % (WR%) in 4 Wochen | 62% |
| Restgrößen in % nach 4 Wochen | 34% |
| Wundflächenreduktion pro Woche in % | 15,5% |
| Medane Wundflächenverkleinerung pro Tag | 0,144 cm²/Tage |
| Dauer zur Heilung pro cm² | 6,92 Tage |
| Abgeheilte Wunden pro Woche | 7,5% |

Einer der Patienten zeigte initial eine besonders große Wundfläche. Wird dieser Patient aus dem Ergebnis ausgeschlossen ergibt sich folgende Auswertung.

**Tabelle 2 zeigt die Ergebnisse für 9 Patienten**

| ALLGEMEINE ERGEBNISGRUNDLAGEN | |
|---|---|
| Bei 10 Patienten durchgeführte Verbandwechsel | 126 |
| Kumulierte Behandlungs- /Heilungstage | 292 Tage |
| Kumulierte Wundflächengrößen | 17,2 cm² |
| Kumulierte Restflächengrößen | 2,6 cm² |
| Gesamtaufwand aller Materialkosten (AEP brutto) | 978,00 € |

| KLINISCHE DATEN | |
|---|---|
| Wundflächenreduzierung in % (WR%) in 4 Wochen | 85% |
| Restgrößen in % nach 4 Wochen | 15% |
| Wundflächenreduktion pro Woche in % | 21,2% |
| Medane Wundflächenverkleinerung pro Tag | 0,059 cm²/Tage |
| Dauer zur Heilung pro cm² | 16,97 Tage |
| Abgeheilte Wunden pro Woche | 8,33% |

### Ergebnis

Der erfindungsgemäße Verband ist geeignet, bei chronischen Wunden eine Wundflächenregression von mehr als 60% in vier Wochen zu erzielen. Figur 2 zeigt eine Abheilungsanalyse. Es zeigt sich, dass bei etwa 50% der Fälle die Abheilungsdauer 8 Wochen beträgt, 75% aller Fälle sind in 12 Wochen abgeheilt.

## Patentansprüche

1. Wundauflage umfassend
eine Absorberschicht aus einem Funktionsvlies umfassend mindestens erste und zweite Filamente,
wobei die ersten Filamente einen Kern aus einem Polyethylenterephtalat und einen Mantel aus Polyolefin umfassen und im Mantel eine Silberquelle eingebunden ist und
wobei die zweiten Filamente einen Kern aus Polyacrylnitril und einen Mantel aus Polyacryl umfassen,
wobei die Absorberschicht in der Lage ist, 0,15 bis 1,20 ml/cm² Wasser zu absorbieren.

2. Wundauflage nach Anspruch 1, **dadurch gekennzeichnet, dass** die Wundauflage mindestens zwei Schichten umfasst und eine wundnahe Schicht aus einem hydrophoben Material mit einer lichten Öffnungsweite von 10 bis 25 µm umfasst.

3. Wundauflage nach Anspruch 2, **dadurch gekennzeichnet, dass** die wundnahe Schicht aus einem Polyolefin, insbesondere Polyethylen besteht.

4. Wundauflage nach mindestens einem der Ansprüche 1 bis 3, **dadurch gekennzeichnet, dass** die zwei Filamente der Absorberschicht zusammen eine Helix bilden.

5. Wundauflage nach mindestens einem der Ansprüche 1 bis 4, **dadurch gekennzeichnet, dass** die Absorberschicht bei Kontakt mit Wasser über einen Zeitraum von 72 Stunden zwischen 10 µg und 100 µg Silberionen pro 100 cm² Wundauflage abgibt.

6. Wundauflage nach mindestens einem der Ansprüche 1 bis 5, **dadurch gekennzeichnet, dass** das erste Filament und das zweite Filament unabhängig voneinander einen Durchmesser von 5 bis 20 µm aufweisen.

7. Wundauflage nach mindestens einem der Ansprüche 1 bis 6, **dadurch gekennzeichnet, dass** das erste Filament und das zweite Filament unabhängig voneinander einen Kern mit einem Durchmesser von 0,5 bis 2 µm aufweisen.

8. Wundauflage nach mindestens einem der Ansprüche 2 bis 7, **dadurch gekennzeichnet, dass** die wundnahe Schicht und die Absorberschicht mit einem Klebstoff verbunden sind.

9. Wundauflage nach mindestens einem der Ansprüche 1 bis 8, **dadurch gekennzeichnet, dass**
• 50 bis 70 % der Absorptionskapazität in 30 sec. aufgenommen werden können und/oder
• die Fähigkeit besteht, mindestens 50 % der Kapazität unter 26,66 mbar (20 mmHg) Kompressionsdruck zurückzuhalten und/oder
• die Querfeuchtigkeit von 5 bis 15 mm in 24 Stunden beträgt.

10. Wundauflage nach mindestens einem der Ansprüche 1 bis 9, **dadurch gekennzeichnet, dass** die Faserfilamente konsistenzbeständig sind.

11. Wundauflage nach mindestens einem der Ansprüche 1 bis 10, **dadurch gekennzeichnet, dass** die ersten und zweiten Filamente in einem Gewichtsverhältnis von 1:3 bis 3:1 von liegen.

12. Wundauflage nach Anspruch 1, bis 11 geeignet zur Behandlung von Wunden, insbesondere Behandlung chronischer Wunden.

13. Kit enthaltend eine Wundauflage nach einem der Ansprüche 1 bis 11 und ein hyaluronsäurehaltiges Spray.

14. Wundauflage nach Anspruch 1 bis 11 geeignet zur Behandlung von Druckgeschwüren (Dekubitus), arteriellem Ulcus, venösen Ulcus, neuropatischem Ulcus, Verbrennungen, insbesondere Verbrennungen vom Grad IIa/IIb, Post OP Wunden, radiologischen Wunden (Strahlenulcera), bakterienverursachten Wunden oder chemisch verursachten Wunden.

15. Verwendung einer Wundauflage nach mindestens einem der Ansprüche 1 bis 11 als Feuchtelektrode, als Waschhandschuh und/oder als Waschpad.

## Claims

1. A wound cover comprising:
an absorber layer made of a functional non-woven comprising at least first and second filaments;
wherein said first filaments comprise a core of a polyethylene terephthalate and a sheath of polyolefin, and a silver source is incorporated in the sheath; and
wherein said second filaments comprise a core of polyacrylonitrile and a sheath of polyacryl;
wherein the absorber layer is capable of absorbing from 0.15 to 1.20 ml/cm² of water.

2. The wound cover according to claim 1, **characterized in that** said wound cover comprises at least two layers including a layer proximal to the wound consisting of a hydrophobic material having an inside opening width of from 10 to 25 µm.

3. The wound cover according to claim 2, **characterized in that** said layer proximal to the wound consists of a polyolefin, especially polyethylene.

4. The wound cover according to at least one of claims 1 to 3, **characterized in that** the two filaments of the absorber layer together form a helix.

5. The wound cover according to at least one of claims 1 to 4, **characterized in that** the absorber layer releases from 10 to 100 µg of silver ions per 100 cm² of wound cover over a period of 72 hours upon contact with water.

6. The wound cover according to at least one of claims 1 to 5, **characterized in that** said first and second filaments independently have a diameter of from 5 to 20 µm.

7. The wound cover according to at least one of claims 1 to 6, **characterized in that** said first and second filaments independently have a core having a diameter of from 0.5 to 2 µm.

8. The wound cover according to at least one of claims 2 to 7, **characterized in that** said layer proximal to the wound and the absorber layer are bonded together with an adhesive.

9. The wound cover according to at least one of claims 1 to 8, **characterized in that**
• from 50 to 70% of the absorption capacity can be taken up within 30 seconds; and/or
• at least 50% of the capacity can be retained under a compressive pressure of 26.66 mbar (20 mm Hg); and/or
• the transverse moisture spread rate is from 5 to 15 mm in 24 hours.

10. The wound cover according to at least one of claims 1 to 9, **characterized in that** said fiber filaments maintain their consistency.

11. The wound cover according to at least one of claims 1 to 10, **characterized in that** said first and second filaments are present in a weight ratio of from 1:3 to 3:1.

12. The wound cover according to claims 1 to 11, suitable for treating wounds, especially for treating chronic wounds.

13. A kit containing a wound cover according to any of claims 1 to 11 and a spray containing hyaluronic acid.

14. The wound cover according to claims 1 to 11, suitable for treating decubitus, arterial ulcer, venous ulcer, neuropathic ulcer, burns, especially burns of degree IIa/IIb, postsurgical wounds, radiological wounds (radiation ulcers), bacterially caused wounds or chemically caused wounds.

15. Use of the wound cover according to at least one of claims 1 to 11 as a wet electrode, as a washing glove, and/or as a washing pad.

## Revendications

1. Pansement, comprenant
une couche absorbante composée d'un non-tissé fonctionnel comprenant au moins des premiers et deuxièmes filaments,
les premiers filaments comprenant un noyau en poly(téréphtalate d'éthylène) et une enveloppe en poly(oléfine), et une source d'argent étant intégrée dans l'enveloppe et
les deuxièmes filaments comprenant un noyau en poly(acrylonitrile) et une enveloppe en poly(acryle),
la couche absorbante étant en mesure d'absorber 0,15 à 1,20 ml/cm² d'eau.

2. Pansement selon la revendication 1, **caractérisé en ce que** le pansement comprend au moins deux couches et comprend une couche proche de la plaie composée d'un matériau hydrophobe ayant une largeur d'ouverture libre de 10 à 25 µm.

3. Pansement selon la revendication 2, **caractérisé en ce que** la couche proche de la plaie se compose de poly(oléfine), en particulier de poly(éthylène).

4. Pansement selon au moins l'une des revendications 1 à 3,**caractérisé en ce que** les deux filaments de la couche absorbante forment ensemble une structure hélicoïdale.

5. Pansement selon au moins l'une des revendications 1 à 4, **caractérisé en ce que** la couche absorbante, lors du contact avec de l'eau, délivre, pendant une période de 72 heures, entre 10 µg et 100 µg d'ions argent par 100 cm² de pansement.

6. Pansement selon au moins l'une des revendications 1 à 5, **caractérisé en ce que** le premier filament et le deuxième filament présentent indépendamment l'un de l'autre un diamètre de 5 à 20 µm.

7. Pansement selon au moins l'une des revendications 1 à 6, **caractérisé en ce que** le premier filament et le deuxième filament présentent indépendamment l'un de l'autre un diamètre de 0,5 à 2 µm.

8. Pansement selon au moins l'une des revendications 2 à 7, **caractérisé en ce que** la couche proche de la plaie et la couche absorbante sont reliées par un adhésif.

9. Pansement selon au moins l'une des revendications 1 à 8, **caractérisé en ce**
• **que** 50 à 70% de la capacité d'absorption peuvent être absorbés en 30 secondes et/ou
• **qu'**il est possible de retenir au moins 50 % de la capacité sous une pression de compression de 26,66 mbar (20 mmHg) et/ou
• **que** l'humidité transversale est de 5 à 15 mm en 24 heures.

10. Pansement selon au moins l'une des revendications 1 à 9, **caractérisé en ce que** les filaments fibreux conservent leur composition.

11. Pansement selon au moins l'une des revendications 1 à 10, **caractérisé en ce que** les premiers et les deuxièmes filaments se situent dans un rapport en poids de 1:3 à 3:1.

12. Pansement selon les revendications 1 à 11, approprié pour le traitement de plaies, en particulier pour le traitement de plaies chroniques.

13. Nécessaire contenant un pansement selon une des revendications 1 à 11 et un spray contenant de l'acide hyaluronique.

14. Pansement selon les revendications 1 à 11, approprié pour le traitement d'escarres (de décubitus), d'ulcères artériels, d'ulcères veineux, d'ulcères neuropathiques, de brûlures, en particulier de brûlures de degré IIa/IIb, de plaies postopératoires, de plaies radiologiques (ulcères dus aux rayons), de plaies causées par des bactéries ou de plaies causées par des produits chimiques.

15. Utilisation d'un pansement selon au moins l'une des revendications 1 à 11 en tant qu'électrode humide, en tant que gant de toilette et/ou en tant que lingette.
